Europäisches Patentamt

**European Patent Office**

Office européen des brevets

(19)

(11) Publication number : **0 549 367 A1**

## (12) EUROPEAN PATENT APPLICATION

(21) Application number : **92311816.0**

(22) Date of filing : **24.12.92**

(51) Int. Cl.⁵ : **A61K 33/06, A61K 33/10, A61K 31/59, A23K 1/16, A23K 1/175**

(30) Priority : **26.12.91 US 814368**

(43) Date of publication of application :
**30.06.93 Bulletin 93/26**

(84) Designated Contracting States :
**BE CH DE DK ES FR GB LI NL**

(71) Applicant : **WISCONSIN ALUMNI RESEARCH FOUNDATION**
**614 North Walnut Street Post Office Box 7365 Madison Wisconsin 53707-7365 (US)**

(72) Inventor : **DeLuca, Hector Floyd**
**1809 Highway BB**
**Deerfield, Wisconsin 53531 (US)**
Inventor : **Hodnett, Dean William**
**2029 9th St. Apt. 3**
**Coralville, Iowa 52241 (US)**
Inventor : **Jorgensen, Neal Albert**
**5979 Woodcreek Lane**
**Middleton, Wisconsin 53562 (US)**

(74) Representative : **Ellis-Jones, Patrick George Armine**
**J.A. KEMP & CO. 14 South Square Gray's Inn London WC1R 5LX (GB)**

(54) **Method of treating milk fever disease in dairy cattle.**

㊄ A method of preventing milk fever disease in dairy cattle which comprises administering to the cattle an active vitamin D compound together with a calcium supplement sufficient to provide at least 250 grams of total calcium per day. The active vitamin D compound is administered in a dosage range of 0.2-500mg depending upon the compound at priodic daily intervals of five days or less, and the calcium supplement is administered daily beginning at least five days prior to calving. The active vitamin D compound is preferably selected from $1\alpha$-OH-$D_3$, $1\alpha$-OH-$D_2$, $1\alpha,25$-$(OH)_2D_3$, $1\alpha,25$-$(OH)_2D_2$, 25-OH-$D_2$, 25-OH-$D_3$ and mixtures thereof. The calcium supplement comprises at least about 100 grams of calcium per day, and comprises a calcium compound selected from calcium salts of carbonate, phosphate, chloride, or acetate.

EP 0 549 367 A1

## Background of the Invention

This invention relates to a method for prophylactically treating dairy cattle for parturient paresis using an active form of vitamin D together with a calcium supplement.

Parturient paresis (milk fever) is a metabolic disease of dairy cows wherein severe hypocalcemia, resulting from parturition and the initial formation of milk, leads to a disorder characterized by the cows' inability to command use of their muscles and a placid, immobile appearance.

The disease is manifested by a decrease in plasma calcium, usually between six to thirty hours after parturition, to a value so low as to induce tetany with resultant immobilization of the cow. There is also generally an accompanying decrease in the blood phosphate level. As an example, the plasma calcium level in a cow prior to calving is about 10 mg/100 ml. (or 10 mg. percent). Following parturition this level will normally dip to about 7-8 mg. percent but will then rise in a reasonable time to the more normal 10 mg. percent range. In a cow afflicted with milk fever, however, after parturition the plasma calcium may dip more drastically, such as into the 5 mg. percent range, and it is recognized that at such plasma calcium levels the cow can go into tetany and a condition known as Downer cow Syndrome.

Such low plasma calcium levels are not necessary in all cases to induce milk fever disease and the disease is experienced at substantially higher calcium levels depending upon the individual animal involved. If treatment for such condition is not, however, immediate and successful there is a real danger that the cow may die or be afflcited with a lasting paralysis, or at the very least that its milk production will be substantially decreased. (See "Milk Fever Causes, Methods of Treatment and Prevention." S. H. Morrison, Vol. 1, No. 2, a publication of Borden Chemical Company, and J. M. Payne, Brit. Vet. Assn. "Recent Advances in Our Knowledge of Milk Fever," presented at 87th Annual Congress of the Association, Sept. 6, 1964).

The incidence of milk fever disease has been estimated to be in the range from about 3.5-5% of the world's dairy cows. In individual herds, however, the incidence may be as high as 60-70%. It appears that the incidence of the disease is highest among high milk producing cows during the third and later lactation periods although at times it has been observed in the second lactation period. In any event, once a cow has had milk fever there is a 50% probability that she will again be so afflicted after a subsequent parturition. As a consequence, there has been much interest in developing procedures for preventing this disorder.

The disease has been clearly related to the low blood calcium resulting from the formation of milk at the time of parturition. However, many animals do not suffer the disease and are able to provide sufficient calcium in their blood from intestinal and bone sources to meet the immediate demands of new milk formation. The reason for the difference among individual animals remains unknown. However, several methods of preventing the disease have been suggested and tried. These are:

(1) calcium infusions, which is the common method of treatment of the disorder;

(2) the administration of large amounts of vitamin D, which will prevent the hypocalcemic response; or

(3) conditioning of animals before parturition by feeding low calcium or acidic diets, or various mineral salts.

Hibbs and co-workers (Hibbs, J. W. and Conrad, H. R., J. Dairy Science 49, 243, 1966) were the first to use very large doses of vitamin D to reduce the incidence of parturient paresis. Currently, one of the most widely used prophylactic for milk fever is the administration of vitamin D in massive dosage. For example, in one method the cow is fed 20 million units per day of vitamin D for three to seven days before calving while in another method 10 million units of vitamin D is injected intramuscularly before calving. Although these methods are of value they are associated with potentially high risk and other disadvantages. With administration of such large dosages of vitamin D there is a real danger of vitamin D toxicity and, as a consequence, death of the cow or damage through abnormal calcification of the soft tissues such as the kidney, aorta, etc. Even if the animal survives without damage the milk produced may not be fit for human or calf consumption for some time because of the high content of vitamin D in the milk. Furthermore, the unpredictability of the calving date places an added difficulty on the farmer as to when the vitamin D dosage should be given. If the vitamin D dosage is given too far in advance the incidence of milk fever disease is actually increased by the treatment.

Calcium infusions are excellent treatments, but it is not practically possible to prevent the disease by calcium infusion alone, since it is not possible to forecast when milk fever will result from calving. Low calcium conditioning of the animal is not desirable since it is during the non-lactating or dry periods that cows must be able to replace the bone calcium which had been depleted by previous lactation. Low calcium treatment therefore limits the lifetime of a usable dairy cow since the dairy cow does not have sufficient time and adequate calcium intake to rebuild skeleton loss during lactation. Often these dairy cattle must be sacrificed after the fourth lactation period. Use of acid diets tends to produce an acidosis that results in other problems. Therefore, none of these treatments has provided a complete solution to the prevention of parturient paresis.

The discovery of active vitamin D metabolites and their analogs has led to the development of more effective methods for treating parturient paresis. Thus, it has been shown that 25-hydroxyvitamin $D_3$, a metabolite

of vitamin D, given in relatively large amounts, provides substantial protection against the disease (U. S. Patent No. 3,646,203). Similarly, much lower doses of $1\alpha$-hydroxyvitamin $D_3$ or 1,25-dihydroxyvitamin $D_3$ have also been shown to be effective in preventing the disease (U. S. patent Nos. 3,879,548 and 4,110,446). Also, the use of a mixture of $1\alpha$-hydroxyvitamin $D_3$ and 25-hydroxyvitamin $D_3$ has been proposed as a method for providing very marked protection against milk fever disease (U. S. patent No. 4,338,312). Such a mixture should not only extend the period of efficacy due to the longer lifetime of 25-hydroxyvitamin $D_3$, but also provide relatively immediate treatment due to the more rapid onset of the action of $1\alpha$-hydroxyvitamin $D_3$.

## Disclosure of the Invention

It has now been found that the combination of an active form of vitamin D together with a calcium supplement sufficient to provide at least a total consumption of 250 grams of calcium per cow per day can be used in the treatment and prevention of milk fever in dairy cattle. Such treatment is more effective than the administration of compounds per se such as vitamin D, 25-hydroxycholecalciferol, $1\alpha$-hydroxycholecalciferol, or the use of a mixture of $1\alpha$-hydroxyvitamin $D_3$ and 25-hydroxyvitamin $D_3$. When the active vitamin D compound is administered at periodic daily intervals of 5 days or less and the calcium supplement administered daily to cows at least about 5 days prior to calving, this combination has been found to prevent the fall in serum calcium andphosphorous characteristically found in cows during the critical period of 24 hours prior to and 48 hours after calving.

As used herein the term "active vitamin D compound" encompasses compounds which control one or more of the various vitamin D-responsive processes in mammals, i.e. intestinal calcium absorption, bone mobilization, and bone mineralization. Thus the vitamin D compounds encompassed by this invention include cholecalcifeiol and ergocalciferol and their known metabolites, as well as the known synthetic cholecalciferol and ergocalciferol analogs which express calcemic activity. These synthetic cholecalciferol and ergocalciferol analogs comprise such categories of compounds as the 5,6-trans-cholecalciferols and 5,6-trans-ergocalciferols, the fluorinated cholecalciferols, the side chain homologated cholecalciferols and side chain homologated $\Delta^{22}$-cholecalciferols. Specific examples of such compounds include vitamin D metabolites or analogs such as vitamin $D_3$, vitamin $D_2$, $1\alpha$-hydroxyvitamin $D_3$, $1\alpha$-hydroxyvitamin $D_2$, $1\alpha$,25-dihydroxyvitamin $D_3$, $1\alpha$,25-dihydroxyvitamin $D_2$, 25-hydroxyvitamin $D_3$, 25-hydroxyvitamin $D_2$, 24,24-difluoro-25-hydroxyvitamin $D_3$, 24,24-difluoro-$1\alpha$,25-dihydroxyvitamin $D_3$, 24-fluoro-25-hydroxyvitamin $D_3$, 24-fluoro-$1\alpha$,25-dihydroxyvitamin $D_3$, $2\beta$-fluoro-25-hydroxyvitamin $D_3$, $2\beta$-fluoro-$1\alpha$-hydroxyvitamin $D_3$, $2\beta$-fluoro-$1\alpha$,25-dihydroxyvitamin $D_3$, 26,26,26,27,27,27-hexafluoro-25-hydroxyvitamin $D_3$, 26,26,26,27,27,27-hexafluoro-$1\alpha$,25-dihydroxyvitamin $D_3$, 24,25-dihydroxyvitamin $D_3$, $1\alpha$,24,25-trihydroxyvitamin $D_3$, 25,26-dihydroxyvitamin $D_3$, $1\alpha$,25,26-trihydroxyvitamin $D_3$, $1\alpha$,25-dihydroxy-24-epi-vitamin $D_2$, 24-homo-1,25-dihydroxyvitamin $D_3$, 24-dihomo-1,25-dihydroxyvitamin $D_3$, 24-trihomo-1,25-dihydroxyvitamin $D_3$ and the corresponding 26- or 26,27-homo, dihomo or trihomo analogs of $1\alpha$, 25-dihydroxyvitamin $D_3$ or $D_2$.

The active vitamin D compound may be made available to a dairy cow either as the sole prophylactic agent or in combination with other active vitamin D compounds or in combination with other agents, such as other therapeutically effective and beneficial substances as may be appropriate for a specific application. In general, dosages of the active vitamin D compound in the range from about 0.2 - 500 mg, depending upon the compound, are effective in preventing milk fever when administered at periodic daily intervals of 5 days or less together with the daily calcium supplement beginning from about 5 days before calving occurs. Dosages should continue until calving. The dose is not critical and can be varied depending at least in part on the size of the animal. In any event, the active vitamin D compound should be administered in amounts sufficient to accomplish the desired treatment or prophylaxis. The use of more than sufficient active vitamin D compound to accomplish the ends should be avoided as an economically unsound practice. It is understood that the specific dosage utilized in any given case will be adjusted in accordance with the specific compounds being employed, the breed of dairy cow to be treated, the condition of the cow and other relevant facts that may modify the activity of the vitamin D compound or the response of the cow as is well known by those skilled in the art. For example, $1\alpha$-hydroxylated vitamin D compounds will be effective at about 0.2-1.0mg dosages whereas 25-hydroxylated vitamin D compounds will be effective at about 4-10mg, and non-hydroxylated compounds at either the 1 or 25 carbon positions, e.g. vitamin $D_2$ and vitamin $D_3$ will be effective at 400-500mg dosages.

The calcium compound employed as the calcium supplement may be in the form of calcium containing compounds i.e. in a combined form such as in calcium salts like calcium carbonate, calcium citrate, calcium phosphate, calcium lactate, calcium acetate, calcium chloride and the like. Combinations of calcium compounds may also be employed. In general, any substance containing calcium that may be metabolized by the dairy cow may be employed as the calcium compound ingredient in the present invention. The amount of calcium compound administered should be sufficient to represent a high calcium supplement for the cow. By "high calcium supplement" as used in this specification it is meant supplementing the cow's normal dietary calcium with additional calcium at a level greater than that level which is normal for the cow. In general, a normal level of calcium would represent about 0.5% to about 1.0% of the cow's diet depending primarily upon the amount of alfalfa (a plant high in calcium content) used in the diet. For example, cows fed a diet consisting of 95% alfalfa silage and 5% grain contains about 1% calcium, and would represent the upper limit of what would be considered a "normal" dietary calcium intake derived from common natural feedstuffs fed to dairy cows. Accordingly, a high calcium supplement for a cow would involve the administration of sufficient calcium to increase the total calcium intake in the cow's diet to represent from about 1.5% to about 3% or more of the diet of the cow. Generally, the calcium supplement would comprise at least about 100 grams and preferably at least about 200 grams of calcium. Such a calcium supplement would provide a total (i.e. the cow's normal dietary calcium together with the calcium supplement) of at least about 250 grams of calcium per day to the cow. However, the proportion of the calcium is dependent upon the particular cow being treated, with the above percentages generally effective to practice the present invention. Although the actual amount of the calcium compound used is not critical in all cases, a sufficient amount of the compound should be used to effect a high calcium supplement for the cow being treated.

Amounts in the diet of the cow in excess of about 3% calcium are generaly unnecessary to achieve the desired results and may not be economically sound practice. In practice, it is understood that the specific dosage utilized in any given case will be adjusted in accordance with the specific compounds being employed, the breed of cow to be treated, the condition of the cow and other relevant facts that may modify the activity of the calcium compound or the response of the cow, as is well known by those skilled in the art.

Effective and practical administration of the vitamin D and calcium compounds can be accomplished by injection of the compounds intravenously, intramuscularly or subcutaneously, while dissolved in a suitable vehicle such as an innocuous oil or propylene glycol. Alternatively, the vitamin D and calcium compounds can be compounded with other materials to form a bolus or can be encapsulated so that it lends itself to oral administration. If desired the materials can be applied topically in a suitable vehicle and perhaps in the presence of an agent e.g. dimethyl-sulfoxide, which enhances penetration of the skin. The dosage forms may also contain adjuvants, such as preserving, stabilizing, wetting or emulsifying agents, solution promoters, binders, fillers, etc. With suitable adjuvants, or in a suitable solvent, administration may also be as a top dressing for grains or other dietary components fed to the animals. A suitable formulation may be provided by microencapsulating the vitamin D compound in oil by standard techniques, or in a vehicle such as polyvinylpropylene and/or gelatine. Suitable antioxidants such as Tenox BHT, a butylated hydroxytoluene (see U. S. Patent No. 3,082,258 to Eastman Kodak) may also be incorporated into the formulation.

The present invention is further described by means of the following illustrative example.

## EXAMPLE

Approximately 50 dairy cattle at the University of Wisconsin managed herd were selected for use based on the criteria that the animals have had a minimum of two previous lactations with approximately a 30% incidence in milk fever disease. These animals were divided into two groups, namely, a control group and a treated group. The treated cattle were given 4 mg of 25-OH-$D_3$ and .5 mg of 1$\alpha$-OH-$D_3$ dissolved in a corn oil vehicle by injection intramuscularly every 5 days beginning with 5 days before the predicted calving date. The calcium and phosphorus levels were taken prior to parturition and after parturition. The animals were given supplemental calcium so that their calcium intake as a percent of the diet was 1.8% calcium and .3% phosphorous. Their total calcium intake, therefore, was 270 grams per day and the phosphorous intake 44 grams per day. The incidence of milk fever disease was 30.8% in the control cattle and was reduced to 8% by the administration of the calcium supplement and vitamin D compounds.

The following table is a comparison of the University of Wisconsin data with data obtained from other field trials. A comparison of the University of Wisconsin data where cows consumed high dietary calcium to the Bayvet field trial data where cows consumed lower dietary calcium indicates that the efficacy of the vitamin D compounds depends upon a high calcium intake. Thus, the utilization of supplemental dietary calcium is of great importance in preventing the hypocalcemia resulting in milk fever disease.

Dietary Calcium, Phosphorus and Milk Fever Incidence
(from Bayvet Field Trial)

| | Estimated Mineral Content of Diet (%) | | Estimated Mineral Intake (g/d) | | Milk Fever Incidence[a] | |
|---|---|---|---|---|---|---|
| | Ca | P | Ca | P | Treated | Control |
| Maddox | 0.53 | 0.37 | 66 | 46 | 5/38 (13) | 4/41 (9.4) |
| Hoffman | 0.67 | 0.29 | 142 | 61 | 8/32 (25) | 3/35 (8.6) |
| Ribeiro | 0.72 | 0.45 | 114 | 71 | 4/10 (40) | 4/10 (40) |
| Bixler | 0.55 | 0.68 | 43 | 53 | 3/9 (33) | 0/5 (0) |
| Wilbur | 0.79 | 0.55 | 97 | 68 | 2/7 (28) | 0/3 (0) |
| Whitney | 0.45 | 0.46 | 53 | 54 | 0/2 (0) | 1/3 (33) |
| Total | | | | | 22/98 (22.4) | 12/97 (12.4) |
| U.W.-Madison | 1.8 | 0.3 | 270 | 44 | 2/24 (8.3) | 8/26 (30.8) |

[a]Number of milk fever cases/total number of cows per group. The number in parentheses is the calculated percent incidence.

It is evident from the data in the foregoing Table that the combination of an active form of vitamin D with a calcium supplement demonstrates a marked ability to prevent the fall in serum calcium and phosphorous levels of cows during and post-calving and is particularly effective in maintaining blood calcium and phosphorous values during the critical period from about 24 hours pre-to 48 hours post-calving thereby preventing parturient paresis.

It is also evident from a comparison of the data in the foregoing Table with the data presented in U. S Patent Nos. 3,646,203 and 3,879,548 relating to the treatment of milk fever with, respectively, 25-hydroxycholecalciferol and 1α-hydroxycholecalciferol, that the benefits afforded by the treatment of this invention are eminently superior.

## Claims

1. An active vitamin D compound and a calcium supplement sufficient to provide at least 250 grams of total calcium per day for use in the treatment of dairy cattle.

2. Use according to claim 1 where the calcium supplement comprises at least about 100 grams of calcium per day.

3. A top dressing for animal feed which contains at least 100 grams of a calcium supplement and an active vitamin D compound for use in the treatment of dairy cattle.

4. Use according to any one of claims 1 to 3 where the calcium supplement comprises at least 200 grams of calcium per day.

5. Use according to any one of claims 1 to 4 where the active vitamin D compound is 1α-OH-D$_3$, 1α,25-(OH)$_2$D$_3$, 1α-OH-D$_2$, 1α,25-(OH)$_2$D$_2$, 25-OH-D$_2$ or 25-OH-D$_3$.

6. Use according to claim 5 where the active vitamin D compound is a mixture selected from $1\alpha$-OH-D$_3$, $1\alpha$-OH-D$_2$, $1\alpha,25$-(OH)$_2$D$_3$, $1\alpha,25$-(OH)$_2$D$_2$, 25-OH-D$_2$ and 25-OH-D$_3$.

7. Use according to any one of the preceding claims where the calcium supplement comprises calcium carbonate, phosphate, chloride or acetate.

8. Use according to any one of the preceding claims where the active vitamin D compound is administered in a range of from 0.2 to 500 mg per day.

9. Use according to claim 7 where the active vitamin D compound is administered in a range of from about 0.2 to 0.5 mg per day.

10. Use according to claim 8 or 9 where the active vitamin D compound is administered five days or less before calving.

11. Use according to claim 8 or 9 where the active vitamin D compound and calcium supplement are administered daily beginning at least five days prior to calving.

12. Use according to any one of the preceding claims where the treatment is by injection.

13. Use according to any one of claims 1 to 9 where the treatment is oral.

| | European Patent Office | **PARTIAL EUROPEAN SEARCH REPORT** which under Rule 45 of the European Patent Convention shall be considered, for the purposes of subsequent proceedings, as the European search report | Application Number EP 92 31 1816 Page 1 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | US-A-4 110 446 (H.F. DELUCA) * column 2, line 37 - line 66 * * column 5, line 27 - line 31; claims * --- | 1-13 | A61K33/06 A61K33/10 A61K31/59 A23K1/16 A23K1/175 |
| X | US-A-4 931 290 (H.J. REBHAN) * column 2, line 45 - line 55 * * column 5, line 6-43; claims * --- | 1-9,13 | |
| X | J. DIARY SCI. vol. 56, no. 7, 1973, pages 889 - 895 W.G. OLSON '25-Hydroxycholecalciferol (25-OHD3) II. Efficacy of parenteral administration in prevention of parturient paresis.' * the whole document * --- -/-- | 1-13 | |
| | | | **TECHNICAL FIELDS SEARCHED (Int. Cl. 5)** A61K A23K |

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search THE HAGUE | Date of completion of the search 24 MARCH 1993 | Examiner ORVIZ DIAZ P. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

# PARTIAL EUROPEAN SEARCH REPORT

Application Number

EP    92 31 1816
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| X | J. DIARY SCI.<br>vol. 60, no. 12, 1977,<br>pages 1910 - 1920<br>D.R. GAST 'Efficacy and safety of 1alpha-hydroxyvitamin D3 for prevention of parturient paresis.'<br>* the whole document * | 1-13 | |
| X | J. DIARY SCI.<br>vol. 62, no. 6, 1979,<br>pages 1009 - 1013<br>D.R. GAST 'Potential use of 1,25-dihydroxycholecalciferol for prevention of parturient paresis.'<br>* the whole document * | 1-13 | |
| P,X | J. DIARY SCI.<br>vol. 75, no. 2, February 1992,<br>pages 485 - 491<br>D.H. HODNETT '1alpha-Hydroxyvitamin D3 plus 25-hydroxyvitamin D3 reduces parturient paresis in dairy cows fed high dietary calcium.'<br>* the whole document * | 1-13 | TECHNICAL FIELDS SEARCHED (Int. Cl. 5) |

EPO FORM 1503 03.82 (P04E10)

MEANINGFUL SEARCH NOT POSSIBLE

1. SUBJECT-MATTER EXCLUDED FROM PATENTABILITY

   Remark : Although claims 2,4-13 are directed to a method
            of treatment of the animal body (Art. 52(4) EPC)
            the search has been carried out. It was based
            on the alleged effects of the compounds.

2. OBSCURITIES, INCONSISTENCIES,

   The expressions "vitamin D compound" and calcium
   supplement" are not sufficient to characterize specific
   chemical compounds. In view of the large number of sub-
   stances encompassed by these terms, the search was limited,
   in principle, to the general inventive idea and to the
   specific compounds mentioned in the claims and in the
   pharmacological example.